# EUROPEAN PATENT SPECIFICATION

(11) **EP 2 275 151 B1**
(45) Date of publication and mention of the grant of the patent: **18.01.2017**
(21) Application number: 09734844.5
(22) Date of filing: 13.04.2009
(51) Int. Cl.: A61L 27/12, A61L 27/24, A61L 27/58, A61L 27/46, C04B 35/447, C04B 40/00

(54) **POWDERY APATITE/COLLAGEN COMPLEX, SHAPE-FORMABLE ARTIFICIAL BONE PASTE, AND METHOD FOR PRODUCTION OF THE COMPLEX**
PULVRIGER APATIT-/COLLAGEN-KOMPLEX, FORMBARE KÜNSTLICHE KNOCHENPASTE UND VERFAHREN ZUR HERSTELLUNG DES KOMPLEXES
COMPLEXE APATITE-COLLAGÈNE POUDREUX, PÂTE D'OS ARTIFICIEL SUSCEPTIBLE D ÊTRE FAÇONNÉE ET PROCÉDÉ DE FABRICATION DU COMPLEXE

(30) Priority: 25.04.2008 JP 2008116348
(43) Date of publication of application: 19.01.2011
(73) Proprietor: Hoya Corporation, Tokyo 161-8525 (JP)
(72) Inventor: SHOJI, Daisuke, Tokyo 161-8525 (JP)
(74) Representative: Schaumburg und Partner Patentanwälte mbB
(86) International application number: PCT/JP2009/057467
(87) International publication number: WO 2009/131026

(56) References cited:
- EP-A1- 0 842 670
- EP-A1- 0 947 489
- EP-A1- 1 642 599
- WO-A1-03/092759
- WO-A1-2004/041320
- WO-A1-2004/103422
- MASANORI KIKUCHI ET AL.: 'Self-organization mechanism in a bone-like hydroxyapatite/ collagen nanocomposite synthesized in vitro and its biological reaction in vivo' BIOMATERIALS vol. 22, 2001, pages 1705 - 1711, XP004245928
- NORIYUKI HISAMORI ET AL.: 'Hone Ruiji Composite no Sosei to Hone Keiseino Hyoka' ORTHOPAEDIC CERAMIC IMPLANTS vol. 23, no. 24, 2005, pages 85 - 93, XP008153422
- YOSHINOBU MANDAI ET AL.: 'Seitaikotsu ni Chikai Seibutsu Tokusei o Motsu Apatite/ Collagen Fukugotaisei Jinkokotsu' KOGYO ZAIRYO vol. 48, no. 11, November 2000, pages 77 - 80, XP008147768

## Description

### FIELD OF THE INVENTION

The present invention relates to an apatite/collagen composite easily filling a small bone defect portion, and a formable-to-any-shape artificial bone obtained by mixing the composite with a binder.

### BACKGROUND OF THE INVENTION

Bone defect portions generated by bruise or illness are now treated by implanting patients' autogenous bones, similar bones provided by others, artificial bones made of metals such as titanium or hydroxyapatite ceramics, etc. Among them, hydroxyapatite ceramics having bone conduction not achieved by conventional metals, polymers or alumina ceramics and directly bonding to bones have been gradually finding wider use as bone-repairing materials substituting autogenous bones in various fields such as oral surgery, neurological surgery, otorhinolaryngology, plastic surgery, etc., since their commercialization. However, artificial bones made of ceramics such as hydroxyapatite are hard and brittle, disadvantageous in difficulty in handling during operation. To solve such problems, apatite/collagen composites having sponge-like elasticity were developed for easy handling. However, filling bone defect portions having complicated shapes and different sizes is still difficult even with such materials, resulting in the likelihood of insufficient filling.

JP 3-128061 A discloses a hydraulic calcium phosphate cement composition comprising as a main component a mixed powder of α-tertiary calcium phosphate and secondary calcium phosphate dihydrate at a Ca/P molar ratio of 1.200-1.498, a hardener for the composition containing at least water-soluble polysaccharide. JP 3-128061 A describes that this cement composition has such proper consistency and good castability that it can fill narrow or complicatedly-shaped portions surely and densely. However, the calcium phosphate cement composition of JP 3-128061 A has poor absorption and replacement by autogenous bone in the living body.

EP 0 842 670 A1 discloses a hydroxyapatite-collagen composite for use as a biomedical material which is a powder form, sheet form or porous form. The hydroxyapatite-collagen composite is being obtained by mixing a collagen solution with hydroxyapatite to form precipitate, freeze-drying the precipitate as necessary, and then sintering at a low temperature with high pressure.

EP 1 642 599 A1 discloses a fibrous apatite/collagen composite having a self-organized structure similar to the structure of a living bone, in which a C-axis is oriented along collagen fibers.

EP 0 947 489 A1 discloses spherical-shape ceramics obtained by dropping calcium phosphate slurry containing a binder from a thin tube into a low temperature refrigerant solution to make a frozen slurry having spherical shape, freeze-drying the spherical frozen slurry, and sintering the dried slurry.

### OBJECT OF THE INVENTION

Accordingly, an object of the present invention is to provide an artificial bone easily absorbed and replaced by autogenous bone without causing any defects when implanted in the living body.

### DISCLOSURE OF THE INVENTION

As a result of intensive research in view of the above object, the inventor has found that powdery apatite/collagen has excellent implantability in bone defect portions having complicated shapes, with good absorption and replacement by autogenous bone. The present invention has been completed based on such finding. In the present invention, a paste comprising powdery apatite/collagen and a binder is preferable.

Thus, the apatite/collagen composite powder of the present invention is absorbed and replaced by autogenous bone in the living body.

The apatite/collagen composite powder is obtained by granulating a blend comprising a fibrous apatite/collagen composite.

The apatite/collagen composite powder preferably has a particle size of 10-2000 µm.

The apatite is low-crystallinity calcium phosphate. The apatite is preferably hydroxyapatite.

The formable-to-any-shape artificial bone paste of the present invention comprises an apatite/collagen composite powder and a binder. The binder is preferably collagen.

### BRIEF DESCRIPTION OF THE DRAWINGS

Fig. 1 is an electron photomicrograph showing an apatite/collagen composite powder produced in Example 3.
Fig. 2 is an electron photomicrograph showing another apatite/collagen composite powder granule produced in Example 3.

### DESCRIPTION OF THE BEST MODE OF THE INVENTION

### [1] Production of powdery (granular) apatite/collagen composite

The formable-to-any-shape artificial bone paste comprises an apatite/collagen composite powder, and a binder such as collagen, etc. The apatite/collagen composite powder is preferably a composite similar to the living bone, in which hydroxyapatite and collagen are orientated in a self-organized manner. The term "self-organized" used herein means that calcium hydroxyphosphate having an apatite structure (hydroxyapatite) has orientation along collagen fibers, peculiar to the living bone; the C-axis of hydroxyapatite being orientated along collagen fibers.

### (1) Starting materials

The apatite/collagen composite is produced from collagen, a phosphate and a calcium salt. The collagen may be extracted from animals, etc., though their kinds, parts, ages, etc. are not particularly restrictive. In general, collagen obtained from skins, bones, cartilages, tendons, internal organs, etc. of mammals such as cow, pig, horse, rabbit and rat and birds such as hen, etc. may be used. Collagen-like proteins obtained from skins, bones, cartilages, fins, scales, internal organs, etc. of fish such as cod, flounder, flatfish, salmon, trout, tuna, mackerel, red snapper, sardine, shark, etc. may also be used. The extraction method of collagen is not particularly restrictive but may be a usual one. In place of collagen extracted from animal tissues, collagen produced by gene recombination technologies may also be used.

The phosphoric acid or its salt [hereinafter simply called "phosphoric acid (salt) "] may be phosphoric acid, disodium hydrogenphosphate, sodium dihydrogenphosphate, dipotassium hydrogenphosphate, potassium dihydrogenphosphate, etc. The calcium salts may be calcium carbonate, calcium acetate, calcium hydroxide, etc. The phosphoric acid and the calcium salt are preferably used in the form of a uniform aqueous solution or suspension.

A mass ratio of apatite to collagen in the apatite/collagen composite is preferably 9/1 to 6/4, more preferably 8.5/1.5 to 7/3, most preferably about 8/2 from the aspect of mechanical strength.

### (2) Preparation of solution

An aqueous solution of collagen and phosphoric acid (salt) is generally prepared by adding an aqueous collagen solution to an aqueous phosphoric acid (salt) solution. The concentration of collagen in the aqueous solution of collagen and phosphoric acid (salt) is preferably 0.1-1.5% by mass, particularly about 0.85% by mass. The concentration of phosphoric acid (salt) is preferably 15-240 mM, particularly about 120 mM. The aqueous collagen solution used preferably contains about 0.85% by mass of collagen and about 20 mM of phosphoric acid. The concentration of an aqueous calcium salt solution (or suspension) is preferably 50-800 mM, particularly about 400 mM. The fiber length of the apatite/collagen composite can be controlled by adjusting the concentration of each solution. Specifically, the higher concentration each solution has, the shorter fibers are obtained, and vice versa.

### (3) Production of apatite/collagen composite

An aqueous solution of collagen and phosphoric acid (salt), and an aqueous calcium salt solution or suspension are simultaneously dropped into water substantially in the same amount as that of the aqueous calcium salt solution or suspension added at about 40°C to form an apatite/collagen composite. The fiber length of the apatite/collagen composite can be controlled by adjusting dropping conditions. The dropping speed is preferably 1 to 60 mL/minute, more preferably about 30 mL/minute. The stirring speed is preferably 1 to 400 rpm, more preferably about 200 rpm. The mixing ratio of the phosphoric acid (salt) to the calcium salt is preferably 1/1 to 2/5, more preferably 3/5. The mixing ratio of collagen to apatite (the total of the phosphoric acid salt and the calcium salt) is preferably 1/9 to 4/6, more preferably 1.5/8.5 to 3/7.

The reaction solution is preferably kept at pH of 8.9 to 9.1 by maintaining a calcium ion concentration at 3.75 mM or less and a phosphoric acid ion concentration at 2.25 mM or less in the reaction solution. Outside the above concentration ranges of the calcium ion and/or the phosphoric acid ion, the self-organization of the composite would be hindered. The above dropping conditions provide the self-organized apatite/collagen composite with fiber length of 2 mm or less suitable as a powdery apatite/collagen material.

### (4) Production of powdery (granular) apatite/collagen composite

The powdery (or granular) apatite/collagen composite can be produced by a method such as freeze drying, granulation.

In the case of the freeze-drying method (not part of the invention), a suspension of the fibrous apatite/collagen composite in a solvent such as water, etc. is sprayed into a liquid (for example, liquid nitrogen) or a gas at -150°C to -250°C by a spray drier, etc. to freeze liquid drops, which are then freeze-dried, and cross-linked by thermal dehydration in vacuum, for example, at 140°C for 12 hours to obtain the apatite/collagen composite powder. The volume ratio of the fibers to the solvent in the suspension is preferably 5/95-1/99. By this method, the fibrous apatite/collagen composite is agglomerated, resulting in substantially spherical apatite/collagen composite powder. Spheroidization makes it easy to extrude a later-described paste comprising the apatite/collagen composite powder and a binder, for example, from a syringe, etc. The particle sizes can be controlled by adjusting a freezing temperature, spraying conditions, etc. in spray-drying.

In the case of a granulation method, a blend of the fibrous apatite/collagen composite with a solvent such as water, etc. is granulated by a wet-extrusion granulator. The mass ratio of the fibers to the solvent is preferably 0.5-1. Cylindrical granules having uneven lengths, which are obtained by wet-extrusion granulation, are preferably spheroidized by a spheroidizing granulator. The resultant spherical particles are dried, and cross-linked by thermal dehydration in vacuum, for example, at 140°C for 12 hours, to obtain substantially spherical apatite/collagen composite powder.
The particle sizes can be controlled by adjusting the mesh size of a screen in the granulator.

The cross-linking may be conducted by using a chemical agent such as glutaraldehyde, etc. The particle size of the apatite/collagen composite powder is preferably 10-2000 µm, more preferably 30-1000 µm. Because of no thermal hysteresis when exposed to high temperatures, apatite is turned to low-crystallinity calcium phosphate (apatite). The apatite/collagen composite powder per se may be used in bone defect portions.

### [2] Formable-in-any-shape apatite/collagen composite

An apatite/collagen composite paste is obtained by mixing the apatite/collagen composite powder, a binder and a physiological saline solution. The binder is preferably collagen. The amount of the binder used is preferably 1-10% by mass based on the apatite/collagen composite powder. The concentration of a solid component (apatite/collagen composite powder and binder) in the paste is preferably 5-10% by volume.

The apatite/collagen composite paste is mixed with an aqueous sodium hydroxide solution to adjust its pH to about 7, so that the binder is turned fibrous, resulting in the hardened composite. Before hardening, the paste can be injected into a bone defect portion by a syringe, etc. for implantation.

The present invention will be explained in more detail referring to Examples below without intention of restricting it thereto.

### Example 1

### (1) Production of apatite/collagen composite fibers

400 ml of a 120-mM aqueous phosphoric acid solution was added to 412 g of aqueous collagen solution containing phosphoric acid (0.97% by mass of collagen, and 20 mM of phosphoric acid), and stirred to obtain a solution I. 400 ml of a 400-mM calcium hydroxide solution (solution II) was also prepared. Both solutions I and II were simultaneously dropped into 200 ml of water (25°C) to obtain a slurry of apatite/collagen composite fibers. The reaction solution was stirred at 200 rpm, and the dropping speed was about 30 ml/min. The amounts of the solutions I and II dropped were adjusted to keep the reaction solution at pH of 8.9-9.1.

### (2) Production of apatite/collagen composite powder (granules) (not part of the invention).

The resultant composite fibers were mixed with water such that the percentage of a liquid was 95% by volume, to prepare a suspension of apatite/collagen composite fibers. The suspension was sprayed into liquid nitrogen at -200°C by a spray drier, and freeze-dried to obtain apatite/collagen composite powder. SEM observation revealed that the powder was constituted by spherical granules having particle sizes of 30-1000 µm.

### (3) Production of formable-to-any-shape apatite/collagen composite

2 g of the apatite/collagen composite powder was mixed with 12.71 ml of a physiological saline solution, and then with 0.1 ml of a 1-N aqueous NaOH solution and stirred to obtain an apatite/collagen composite dispersion. This dispersion was mixed with 3.34 g of an aqueous collagen solution containing phosphoric acid (0.58% by mass of collagen and 20 mM of phosphoric acid), and stirred to obtain a viscous (flowable), formable-to-any-shape paste of the apatite/collagen composite. The paste had ionic strength of about 8 and pH of about 7. The amount of a liquid (a physiological saline solution, an aqueous phosphoric acid solution and an aqueous NaOH solution) in the paste was 95% by volume.

This paste was charged into a syringe having a needle diameter of 2.1 mm, and could be extruded from the syringe smoothly. The extruded paste was heated and kept at 37.5°C, forming a gel-like hardened body without fluidity in 120 minutes.

### Example 2

2 g of the apatite/collagen composite powder obtained in the step (2) in Example 1 was mixed with 14.09 ml of a physiological saline solution, and then with 0.06 ml of a 1-N aqueous NaOH solution and stirred to prepare an apatite/collagen composite dispersion. This dispersion was mixed with 2.00 g of an aqueous collagen solution containing phosphoric acid (0.97% by mass of collagen and 20 mM of phosphoric acid), and stirred to prepare a viscous (flowable), formable-to-any-shape paste of the apatite/collagen composite. The paste had ionic strength of about 8 and pH of about 7. The amount of a liquid (physiological saline solution, an aqueous phosphoric acid solution and an aqueous NaOH solution) in the paste was 95% by volume.

This paste was charged into a syringe having a needle diameter of 2.1 mm, and could be extruded from the syringe smoothly. The extruded dispersion was heated and kept at 37.5°C, forming a gel-like (no flowability) hardened body in 120 minutes. The paste of Example 2 had higher flowability in extrusion than that of Example 1.

### Example 3

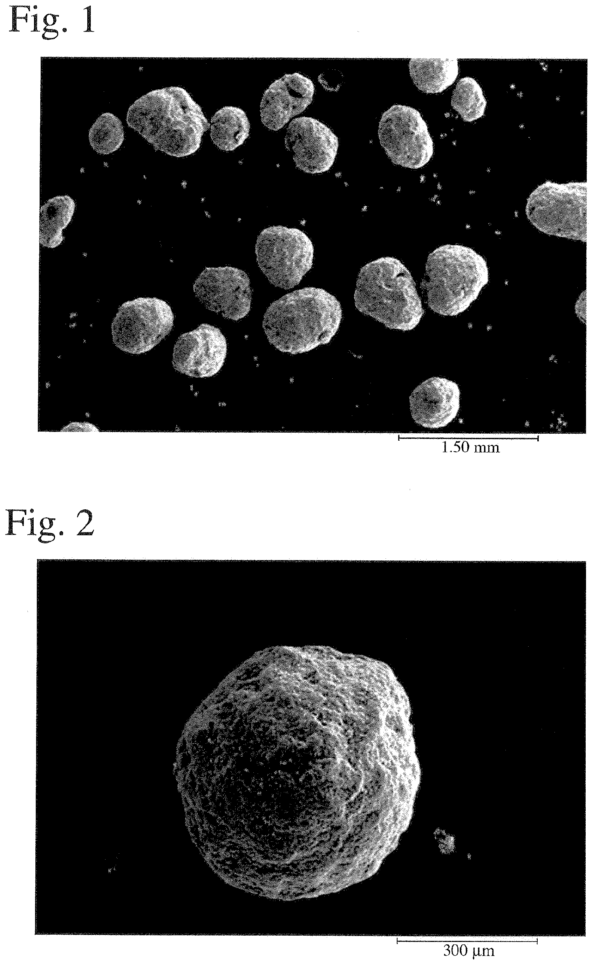

30 g of the apatite/collagen composite fibers obtained in the step (1) in Example 1 was mixed with of 35 g of a physiological saline solution and blended. This blend was granulated by a wet-extrusion granulator (screen mesh diameter: 0.7 mm, rotation speed: 60 rpm, and load current: 2.2 A). Cylindrical granules with uneven lengths obtained by wet-extrusion granulation were spheroidized by a spheroidizing granulator (plate: 3 mm, and rotation speed: 600 rpm), dried, and cross-linked to obtain an apatite/collagen composite powder. SEM observation revealed that this apatite/collagen composite powder was constituted by spherical particle having diameters of 300-500 µm as shown in Figs. 1 and 2.

### Using the apatite/collagen composite powder, a

formable-to-any-shape apatite/collagen composite paste was produced in the same manner as in Example 2. This paste was extruded from the syringe in the same manner as in Example 2 to evaluate its extrudability and hardenability.
As a result, the same results as in Example 2 were obtained.

### EFFECT OF THE INVENTION

The apatite/collagen composite powder of the present invention can easily provide a formable-to-any-shape artificial bone paste. Because the artificial bone paste of the present invention has excellent implantability in bone defect portions having complicated shapes and different sizes, as well as good absorption and replacement by autogenous bone, it can be implanted in any portion regardless of its shape, and has excellent biocompatibility.
Accordingly, burden is reduced for a patient having the artificial bone implanted, and the artificial bone need not be worked for implanting, enabling a doctor to use it efficiently.

## Claims

1. A spherical apatite/collagen composite powder, which is absorbed and replaced by autogenous bone in the living body, obtained by granulating a blend comprising a fibrous apatite/collagen composite, wherein said apatite is low crystallinity calcium phosphate.

2. The spherical apatite/collagen composite powder according to claim 1, which has a particle size of 10-2000 µm.

3. The spherical apatite/collagen composite powder according to claim 1, wherein said apatite is hydroxyapatite.

4. A formable-to-any-shape artificial bone paste, which comprises the spherical apatite/collagen composite powder recited in claim 1 and a binder.

5. The formable-to-any-shape artificial bone paste according to claim 4, wherein said binder is collagen.

## Patentansprüche

1. Sphärisches Apatit-Kollagen-Kompositpulver, das im lebenden Körper absorbiert und durch autogenen Knochen ersetzt wird, erhalten durch Granulieren einer Mischung, die faseriges Apatit-Kollagen-Komposit enthält, wobei der Apatit Kalziumphosphat geringer Kristallinität ist.

2. Sphärisches Apatit-Kollagen-Kompositpulver nach Anspruch 1, das eine Teilchengröße von 10-2000 µm hat.

3. Sphärisches Apatit-Kollagen-Kompositpulver nach Anspruch 1, wobei der Apatit Hydroxylapatit ist.

4. Beliebig verformbare Kunstknochenpaste, die ein sphärisches Apatit-Kollagen-Kompositpulver nach Anspruch 1 und einen Binder enthält.

5. Beliebig verformbare Kunstknochenpaste nach Anspruch 4, wobei der Binder Kollagen ist.

## Revendications

1. Poudre composite collagène/apatite sphérique, qui est absorbée et remplacée par un os autogène dans le corps vivant, obtenue par granulation d'un mélange comprenant un composite collagène/apatite fibreux, dans lequel ladite apatite est un phosphate de calcium à
faible cristallinité.

2. Poudre composite collagène/apatite sphérique selon la revendication 1, qui a une dimension des particules de 10 à 2000 µm.

3. Poudre composite collagène/apatite sphérique selon la revendication 1, dans laquelle ladite apatite est de l'hydroxyapatite.

4. Pâte d'os artificiel pouvant prendre une forme quelconque, qui comprend la poudre composite collagène/apatite sphérique exposée dans la revendication 1 et un liant.

5. Pâte d'os artificiel pouvant prendre une forme quelconque selon la revendication 4, dans laquelle ledit liant est du collagène.
